(19) Europäisches Patentamt
European Patent Office
Office européen des brevets

(11) Numéro de publication: 0 025 727
B1

(12) # FASCICULE DE BREVET EUROPEEN

(45) Date de publication du fascicule du brevet: 28.03.84

(21) Numéro de dépôt: 80401180.7

(22) Date de dépôt: 13.08.80

(51) Int. Cl.³: C 07 D 209/14, C 07 D 209/16, A 61 K 31/40

(54) **Dérivés de l'indole, procédé pour leur préparation et médicaments les contenant.**

(30) Priorité: 17.08.79 FR 7920907

(43) Date de publication de la demande: 25.03.81 Bulletin 81/12

(45) Mention de la délivrance du brevet: 28.03.84 Bulletin 84/13

(84) Etats contractants désignés: BE CH DE GB IT LI NL SE

(56) Documents cités:
GB - A - 2 001 633

CHEMICAL ABSTRACTS, vol. 64, (1966) ref. 2041e,f Columbus, Ohio, US G.B. JACKMAN et al.: "Some tryptamine derivatives: 1-aryloxy-3-(2-indol-3-ylethyl)-amino-propan-2-ols"
Progrés des recherches pharmaceutiques, Vol. 20, p. 226-230, (1976)
E.J. Ariens, Drug Design, Vol. III, p. 216-217, (1972)

Le dossier contient des informations techniques présentées postérieurement au dépôt de la demande et ne figurant pas dans le présent fascicule.

(73) Titulaire: SANOFI, société anonyme
40, Avenue George V
F-75008 Paris (FR)

(72) Inventeur: Demarne, Henri
Le Florence - avenue Major Flandre
F-34000 Montpellier (FR)
Inventeur: Wagnon, Jean
195 rue Michel Ange
F-34000 Montpellier (FR)

(74) Mandataire: Combe, André et al,
CABINET BEAU DE LOMENIE 55 rue d'Amsterdam
F-75008 Paris (FR)

*Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).*

Courier Press, Leamington Spa, England.

EP 0 025 727 B1

Dérivés de l'indole, procédé pour leur préparation et médicaments les contenant.

La présente invention concerne en tant que produits nouveaux des substances chimiques dérivées de l'indole ainsi que leurs sels d'addition d'acides et les isomères desdits dérivés.

L'invention concerne également un procédé de préparation ainsi que leur application en thérapeutique.

Le brevet GB—2,001,663 décrit des composés analogues aux composés de l'invention, dérivés de diméthyltryptamine et possédant des proprietés $\beta$-bloquantes non cardiosélectives.

Les composés selon l'invention sont choisis parmi l'ensemble constitué par:

a) les composés répondant à la formule générale:

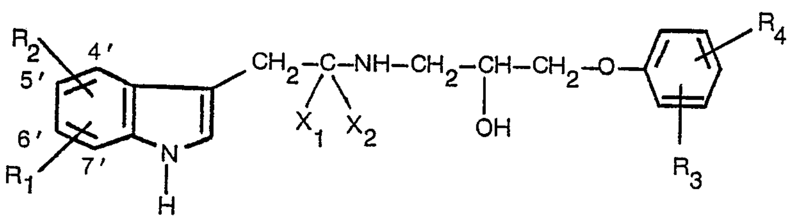

(I)

dans laquelle:

$R_1$ et $R_2$ désignent chacun indépendamment un atome d'hydrogène ou un atome d'halogène, un groupe alkyle en $C_1$—$C_5$, un groupe alcoxy en $C_1$—$C_5$, occupant l'une des positions 4′ à 7′ du noyau indole;

$R_3$ et $R_4$ considérés indépendamment désignent un atome d'hydrogène, un alkyle en $C_1$—$C_5$, un alcoxy en $C_1$—$C_5$, un atome d'halogène, un groupe nitro, un groupe acyle $R_5CO$, un groupe alkylthio $R_5S$ ou alkylsulfinyl $R_5SO$ ou alkylsulfonyl $R_5SO_2$ dans lesquels $R_5$, désigne un groupe alkyle en $C_1$—$C_5$ ou cyclopropyle ou phényle;

$R_3$ et $R_4$ peuvent également désigner un groupe $(CH_2)_nCOOR_6$ ou $CH_2CONH_2$ ou $(CH_2)_nCONH$—$R_6$ ou $(CH_2)_nNHCOOR_6$ ou $(CH_2)_nNHCOR_6$ dans lesquels n représente un entier de 0 à 2 et $R_6$ désigne un groupe alkyle en $C_1$—$C_5$;

enfin $R_3$ et $R_4$ pris ensemble peuvent former un cycle comportant éventuellement 1 hétéroatome de façon à constituer, avec le cycle benzénique auquel ils sont liés, un noyau bicyclique choisi parmi les noyaux suivants:

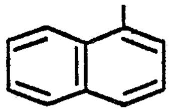

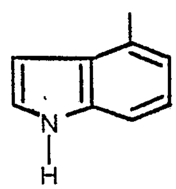

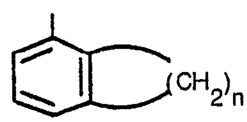

n = 3 à 5

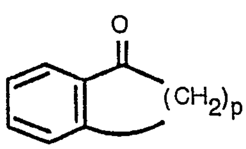

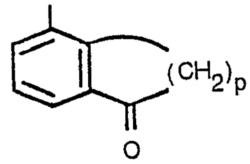

p = 2 à 4

$X_1$ représente un atome d'hydrogène, un groupe alkyle en $C_1$—$C_5$, un groupe hydroxyméthyle ou encore un groupe $COOR_7$ dans lequel $R_7$ représente l'hydrogène ou un groupe alkyle in $C_1$—$C_5$;

$X_2$ représente l'hydrogène ou dans le cas où

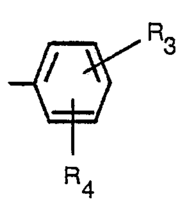

représente un noyau bicyclique autre que naphtyl, $X_2$ peut être un groupe méthyle.

b) les sels d'addition que les composés I sont susceptibles de donner avec les acides minéraux ou organiques pharmaceutiquement acceptables tels que l'acide chlorhydrique, l'acide citrique, l'acide maléique, l'acide fumarique, l'acide tartique.

Dans le présent brevet, on entend par groupe alkyle inférieur, un groupe alkyle droit ou ramifié comportant de 1 à 5 atomes de carbone.

Les composés I, lorsque

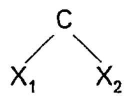

n'est pas un carbone asymétrique, ne comportent qu'un seul atome de carbone asymétrique au niveau de la fonction alcool et peuvent donc exister sous forme de 2 isomères optiques R et S. Lorsque

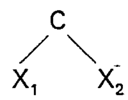

est lui-même un atome de carbone asymétrique, les composés I possèdent 2 centres d'asymétrie et par suite il existe 4 stéréoisomères: RR, RS, SR, et SS. Aussi bien les isomères optiques que les stéréoisomères font partie intégrante de l'invention.

Les composés I peuvent être obtenus selon le schéma réactionnel suivant:

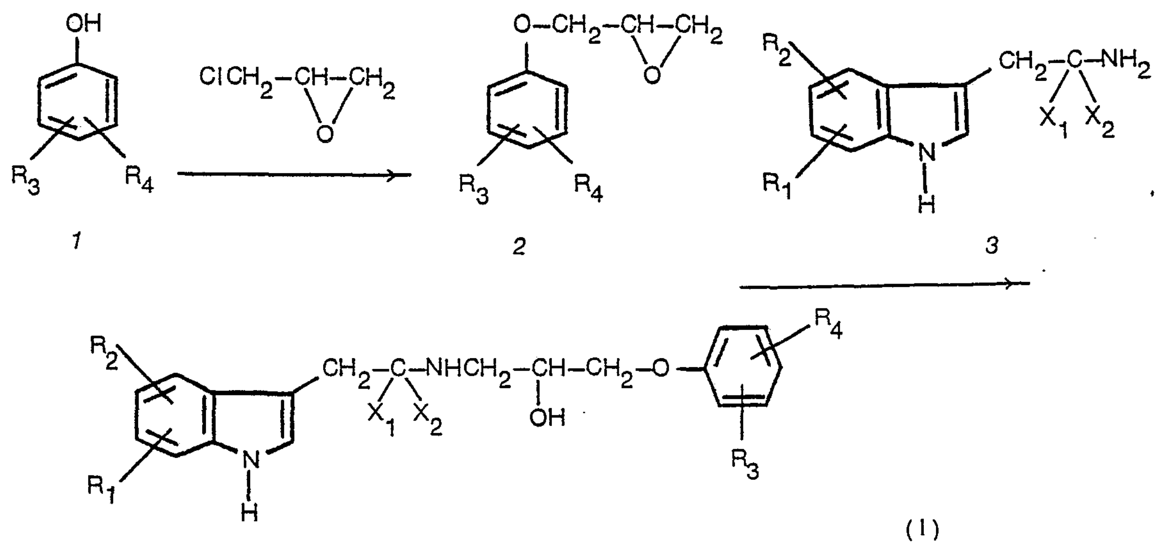

1 2 3

(I)

Par action de l'épichlorhydrine sur un phénol convenablement substitué *1*, au sein d'un solvant anhydre tel l'éthanol au reflux et en présence d'un agent alcalin, on obtient l'époxyde *2* qui est ouvert par action d'une amine *3* convenablement choisis au sein d'un solvant anhydre tel que l'éthanol.

Lorsque

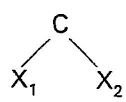

n'est pas un carbone asymétrique on obtient les produits I sous forme de mélange des isomères optiques R et S qu'il est possible de séparer par les méthodes classiques de dédoublement en particulier par combinaison au sein d'un solvant convenable avec un acide optiquement actif tel que les acides D et L tartriques ou D et L benzoyl tartriques.

Lorsque

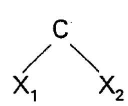

est un atome de carbone asymétrique, la réaction conduit à 4 stéréoisomères. En utilisant une amine *3* préalablement séparée en ses isomères $R_1$ et $S_1$, il est possible d'obtenir directement à partir du

racémique de *2* ($RS_2$) chacun des 2 isomères ($RS_2$)—$R_1$ et ($RS_2$)—$S_1$. Chacun de ces isomères peut ensuite être dédoublé par les méthodes classiques de cristallisations fractionnées et conduit aux 4 isomères: $R_2R_1$, $S_2R_1$, $R_2S_1$, $S_2S_1$.

Les composés dans lesquels $X_1 = CH_3$, $X_2 = H$ peuvent également s'obtenir à partir d'un acétonyl indole et d'un phénoxy-1 amino-3 propanol-2:

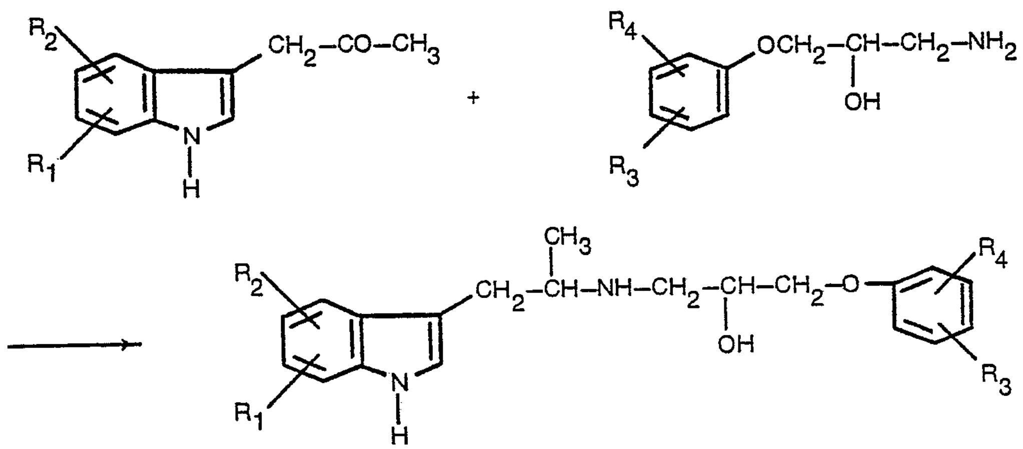

La condensation a lieu par mélange des réactifs au sein d'un solvant tel qu'un alcool aliphatique inférieur à température peu élevée et de préférence à température ambiante. L'imine formée intermédiaire est réduite sans isolement par un agent réducteur tel que le borohydrure de sodium.

Dans le cas de dérivés du tryptophanol ($X_1 = CH_2OH$, $X_2 = H$), il est possible d'effectuer une synthèse stéréosélective des 4 isomères des composés I. On sait, en effet, que la réduction d'un isomère optique du tryptophane conduit au tryptophanol optiquement actif.

Ainsi le L-tryptophane conduit au S-tryptophanol et inversement le D-tryptophane fournit le R-tryptophanol. (Biochimica Biophysica Acta *341,* 284, 1974).

Par condensation de l'un des isomères optiques avec un époxyde *2* racémique, on obtient un mélange de composés $S_1R_2 + S_1S_2$ à partir du S-tryptophanol et $R_1R_2 + R_1S_2$ à partir du R-tryptophanol.

A partir de ces mélanges on peut séparer par cristallisation chacun des isomères à l'état pur.

Enfin, dans le cas particulier des dérivés du tryptophanol où $R_4 = CH_3$ en ortho et $R_5 = H$, il est possible de synthétiser directement les 4 isomères de (I).

En effet, il est connu (J. Chem. Soc. Comm. *1973*, 896) que le diol:

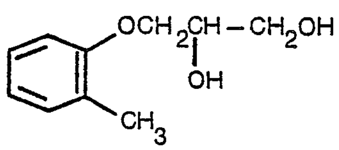

peut être séparé en ses isomères R et S.

Chacun des isomères, tosylé sur la fonction alcool primaire, est condensé avec chacun des isomères du tryptophanol par chauffage au sein d'un solvant inerte tel que l'acétonitrile. On obtient ainsi les 4 isomères du composé I correspondant.

Les exemples suivants illustrent l'invention sans en limiter la portée.

Exemple 1.

*N-[(chloro2 propionyl-4 phénoxy)-3 hydroxy-2 propyl] (amino-2 propyl)-3 indole, fumarate acide* (CM 7743)

(I) $R_1 = R_2 = H$; $X_1 = CH_3$; $X_2 = H$; $R_3 = 2—Cl$; $R_4 = 4—COCH_2CH_3$

1 — (chloro-2 propionyl-4 phénoxy)-1 époxy-2,3 propane

On dissout 2,5 g de chloro-2 propionyl-4 phénol et 10 ml d'épichlorhydrine dans 20 ml d'éthanol absolu. On ajoute 0,9 g de soude et porte au reflux pendant 2 h.

On ajoute au mélange 50 ml d'eau puis on extrait avec du chlorure de méthylène. On lave la phase organique avec de l'eau, sèche sur sulfate de sodium et évapore à siccité.

On obient 3 g du produit attendu utilisé tel quel pour l'opération suivante.

2 — CM 7743

On dissout l'époxyde obtenu ci-dessus (3 g) et 2 g d'(amino-2 propyl)-3 indole dans 50 ml d'éthanol absolu. On chauffe au reflux pendant 6 h puis on évapore le solvant. Le produit brut ainsi

obtenu dissous dans l'acétate d'éthyle est passé sur une colonne de silice. En éluant par un mélange méthanol-acétate d'éthyle (10—90 vol/vol) on obtient la base pure (2,85 g).

Cette base dissoute dans un mélange éthanol acétone est traitée à chaud par une quantité équimoléculaire d'acide fumarique. Par refroidissement le fumarate neutre cristallise; F: 118—122°C.

Exemple 2

*N-[(méthyl-2 phénoxy)-3 hydroxy-2 propyl] DL tryptophane.* CM 7897

(I) $R_1 = R_2 = H$; $X_1 = COOH$; $X_2 = H$; $R_3 = 2 - CH_3$; $R_4 = H$

On dissout, par chauffage, 8,7 g de DL tryptophane et 1,7 g de soude dans 30 ml de diméthylformamide. A la solution, on ajoute 7 g de (méthyl-2 phénoxy)-1 époxy-2,3 propane et porte à ébullition pendant 3 h.

On évapore le solvant sous vide. On reprend le résidu par 200 ml d'eau et ajuste le pH à 7. Il se forme un précipité qu'on essore et lave 2 fois avec du méthanol bouillant. On obtient des cristaux incolores (4,5 g); F: 220—221°C.

En opérant selon le procédé des exemples 1 ou 2 mais en faisant varier les substituants $R_1$, $R_2$, X, $R_3$, $R_4$ et $R_5$, on obtient les produits (I) rassemblés dans le tableau I ci-après.

De même, en remplaçant l'époxyde monocyclique de départ par un époxyde résultant d'un phénol bicyclique, on obtient les composés (I) rassemblés dans le tableau II ci-après.

Exemple 3

*N-(phénoxy-3 hydroxy-2 propyl) (amino-2 propyl)-3 indole.* CM 7783

(I) $R_1 = R_2 = H$; $X_2 = CH_3$; $X_2 = H$; $R_3 = R_4 = H$

On chauffe au reflux, pendant une nuit, le mélange de 2,9 g de phénoxy-3 hydroxy-2 propylamine et 3 g d'acétonyl-3 indole dans 75 ml d'éthanol absolu.

On refroidit la solution dans un bain de glace et on ajoute lentement une suspension de 4 g de borohydrure de sodium dans 10 ml d'un mélange eau-éthanol (50/50 vol/vol). On laisse sous agitation pendant 2 h 30 à température ambiante puis on détruit l'excès de borohydrure par addition d'acide acétique dilué.

On évapore à siccité et on reprend le résidu par de la soude diluée. On extrait 3 fois avec de l'acétate d'éthyle, on lave la solution avec de l'eau et on sèche sur sulfate de sodium. On chromatographie sur colonne de gel de silice. En éluant par un mélange acétate d'éthyle-méthanol (7/3 vol/vol), on obtient un produit qui cristallise. Poids: 2,75 g.

Après 2 recristallisations dans le mélange chlorure de méthylène-éther isopropylique, F: 124—126°C.

Exemple 4

*RS( — ) N-[(méthyl-2 phénoxy)-3 hydroxy-2 propyl]tryptophanol.* CM 7903

(I) $R_1 = R_2 = H$; $X_1 = CH_2OH$; $X_2 = H$; $R_4 = 2\text{-}CH_3$; $R_5 = H$

1 — S( + ) (méthyl-2 phénoxy)-1 toxyloxy-3 propanol-2.

On dissout 5,5 g de R (méthyl-2 phénoxy)-1 propane diol-2,3 dans 25 ml de pyridine sèche, et on refroidit la solution à −13°C. On ajoute ensuite par petites fractions en 30 min, 5,75 g de chlorure de para-toluène sulfonyle fraîchement recristallisé. On laisse le mélange pendant 8 jours à 0°C. On ajoute alors de l'acétate d'éthyle (100 ml) puis sous agitation à froid une solution d'acide sulfurique à 20% (150 ml). On sépare la phase organique puis réextrait 3 fois la phase aqueuse avec de l'acétate d'éthyle. On réunit les extraits organiques, on lave avec de l'eau jusqu'à neutralité et on sèche sur sulfate de sodium. On évapore à siccité à 50°C sous vide.

L'huile résiduaire est chromatographiée sur colonne de gel de silice. On élue le tosylate par de l'éther pur et on obtient un produit huileux (7,85 g) $\alpha_D^{26} = +12,8$ (C = 8,39; chloroforme).

2 — CM 7903

On chauffe au reflux, pendant 26 h, le mélange de 3,8 g du tosylate précédent et 4,3 g de S(—) tryptophanol dans 70 ml d'acétonitrile.

On évapore à siccité puis on reprend le résidu dans l'acétate d'éthyle. On agite cette solution pendant 2 h à température ordinaire avec une solution de soude diluée. On sépare la phase organique et on réextrait 3 fois la phase aqueuse avec de l'acétate d'éthyle. On réunit les extraits organiques, on lave avec de l'eau et on sèche sur sulfate de sodium.

L'huile ainsi obtenue est chromatographiée sur une colonne de gel de silice. Avec un mélange acétate d'éthyle-méthanol (90/10 vol/vol) on élue un produit huileux encore impur.

On le purifie par formation du fumarate dans le mélange éthanol-acétate d'éthyle. Le fumarate cristallisé est décomposé par la soude et fournit une base cristallisée. On recristallise 2 fois dans un mélange dichlorométhane-éther isopropylique on obtient finalement un solide (1,2 g); F: 107—109°C; $\alpha_{589}^{25,5} = -17$; $\alpha_{500}^{25,5} = -30$; $\alpha_{350}^{25,5} = -97$ (C = 0,6; méthanol)

Exemple 5

*SS(—) N-[(méthyl-2 phénoxy)-3 hydroxy-2 propyl] tryptophanol.* CM 7898

1 — R( — ) (méthyl-2 phénoxy)-1 tosyloxy-3 propanol-2.

On opère comme dans l'exemple 4.1- en remplaçant le R (méthyl-2 phénoxy)-1 propane diol-2,3 par l'isomère S.

De la même façon on obtient le produit attendu sous forme d'huile; $\alpha_D^{26} = -12,4$ (C = 8,0; chloroforme).

2 — CM 7898

On opère comme dans l'exemple 4.2- en remplaçant l'isomère S( + ) du tosylate par l'isomère R( — ) obtenu ci-dessus.

De la même façon on obtient CM 7898.

F: 150—151°C; $\alpha_{589}^{25,5} = -12,8$; $\alpha_{500}^{25,5} = -19,5$; $\alpha_{350}^{25,5} = -54,5$ (C = 0,6; méthanol).

Exemple 6

En opérant comme les exemples 4 et 5 mais en remplaçant le S( — ) tryptophanol par le R( + ) tryptophanol, on obtient de la même façon;

— le RR( + ) N-[(méthyl-2 phénoxy)-3 hydroxy-2 propyl] tryptophanol (CM 7902); F: 150,5—151,5°C;

$\alpha_{589}^{25,5} = +14$; $\alpha_{500}^{25,5} = +19$; $\alpha_{350}^{25,5} = +54$ (C = 0,6; méthanol),

— et le SR( + ) N-[(méthyl-2 phénoxy)-3 hydroxy-2 propyl]tryptophanol (CM 7899), F: 107—108°C;

$\alpha_{589}^{25,5} = +18,2$; $\alpha_{500}^{25,5} = +28$; $\alpha_{350}^{25,5} = +96$ (C = 0,6; méthanol).

Exemple 7

*RS( + ) et SS( — )* N[(méthyl-2 phénoxy)-3 hydroxy-2 propyl]tryptophanol. CM 7903 et CM 7898.

On chauffe au reflux pendant 2 h, 3,35 g de S( — ) tryptophanol et 2,9 g de (méthyl-2 phénoxy)-1 époxy-2,3 propane racémique dans 70 ml d'éthanol. Par concentration du solvant sous vide, on obtient un solide cristallisé que l'on essore et recristallise 3 fois dans un mélange méthanol-acétate d'éthyle.

On obtient finalement 0,65 g de cristaux; F: 150—151,5°C; $\alpha_{589}^{25,5} = -13,5$; $\alpha_{500}^{25,5} = -21,5$; $\alpha_{350}^{25,5} = -56$; (C = 0,6; méthanol).

Ce produit est identique à celui de l'exemple 5: CM 7898.

Les solutions mères de la cristallisation et de la 1ére recristallisation sont réunies et chromatographiées sur gel de silice comme indiqué dans l'exemple 4.

Le solide obtenu est purifié par formation puis décomposition du fumarate (voir exemple 4) et conduit finalement après 2 recristallisations dans le mélange de dichlorométhane-éther isopropylique à 0,69 g de cristaux; F: 106—107°C.

$\alpha_{589}^{25,5} = -17$; $\alpha_{500}^{25,5} = -27$; $\alpha_{350}^{25,5} = -96,5$;(C = 0,62; méthanol).

Ce produit est identique à celui obtenu dans l'exemple 4: CM 7903.

Les produits de l'invention ont été étudiés en vue de déterminer leur activité pharmacologique et plus spécialement leur activité sur le système cardiovasculaire.

Les produits de l'invention ont été soumis aux épreuves pharmacodynamiques indiquées ci-dessous.

*Action pharmacologique in vivo chez le chien.*

Le chien est anesthésié au pentobarbital sodique, administré par voie intraveineuse à la dose de 30 mg/kg. Une canule placée dans la veine saphène permet les injections intraveineuse des produits. L'animal est intubé et laissé en respiration spontanée.

La fréquence cardiaque et la pression artérielle systémique sont étudiées et les variations de ces paramètres sont observées après injection intraveineuse du produit à tester, chaque produit étant testé à doses croissantes.

— Antagonisme des effets de l'isoprénaline.

L'antagonisme des produits vis-à-vis des effets cardiovasculaires β stimulants de l'isoprénaline sur les récepteurs β adrénergiques a été recherché. Les résultats sont présentés dans le tableau III ci-après et exprimés en $DI_{50}$: il s'agit de la dose exprimée en mg/kg qui provoque l'inhibition de 50% de la tachycardie ($\beta_1$) et de l'hypotension ($\beta_2$) induite par l'isoprénaline administrée par voie intraveineuse. Dans la dernière colonne est indiquée la cardiosélectivité ($\beta_1 > \beta_2$) ou une faible cardiosélectivité $\beta_1 \geqslant \beta_2$, ou aucune cardiosélectivité $\beta_1 < \beta_2$ et $\beta_1 = \beta_2$.

— Antagonisme des effets de la noradrénaline.

L'antagonisme des produits vis-à-vis des effets vasculaires provoqués par l'administration intraveineuse de noradrénaline sur les récepteurs α adrénergiques a été recherché. Les résultats présentés dans le tableau III sont exprimés en $DI_{50}$: c'est la dose (mg/kg) qui provoque l'inhibition de 50% de la réponse pressive due à l'administration intraveineuse de noradrénaline.

*Action pharmacologique in vitro.*

Tests de "Binding".

Les tests de binding sont effectués sur des suspensions de membranes plasmatiques provenant de divers organes. Les suspensions de membranes sont mises à incuber avec le ligand tritié et diverses concentrations de produits à tester. A l'issue de l'incubation, chaque essai est filtré sur filtre GFB (Whatman), les filtres sont séchés et introduits dans des fioles de comptage. La mesure de la radioactivitè est évaluée par scintillation en milieu liquide. Les résultats sont exprimés en $CE_{50}$ de déplacement du ligand tritié, c'est-à-dire la concentration du produit qui déplace 50% de la radioactivité du ligand.

— Pouvoir de déplacement du $^3H$ dihydroalprénolol.

Les essais ont été effectués sur des membranes plasmiques de coeurs de chiens (récepteurs $\beta_1$) et de poumons de rats (récepteurs $\beta_2$), en présence de $^3H$ dihydroalprénolol $4 \times 10^{-9}M$.

— Pouvoir de déplacement de la $^3H$ clonidine, et du $^3H$ WB 4101.

Les essais ont été effectués sur des membranes plasmiques de cerveaux de rat, en présence de $^3H$ clonidine $1,2 \times 10^{-9}M$ (récepteur $\alpha_2$) et de $^3H$ WB 4101 N-[2-(2,6-diméthoxyphénoxy)éthyl]-1,4-benzodioxane-2-méthylamine, $0,2 \times 10^{-9}M$ (récepteur $\alpha_1$).

Les résultats concernant divers produits selon l'invention sont rassemblés dans le tableau III. En fin de tableau figurent, à titre de témoins, d'une part le propanolol dont l'activité $\beta$-bloquante est bien connue et d'autre part un produit de structure assez voisine des produits de l'invention inclus dans la formule générale du brevet belge no. 868,943.

CM 7854: désigne le N-[(méthyl-2 phénoxy)-3 hydroxy-2 propyl] (amino-2 méthyl-2 propyl)-3 indole.

Ces résultats montrent que les composés selon l'invention présentent une activité inhibitrice importante sur les récepteurs $\beta$ adrénergiques, activité souvent supérieure à celle du propanolol.

Certains de ces produits sont cardiosélectifs in vivo (inhibent de façon sélective les récepteurs cardiaques: $\beta_1$, test à l'isoprénaline), ce sont les CM: 7744, 7748, 7782, 7798, 7808, 7859, 7983 et 40038. Cette note cardiosélective in vivo a été confirmée in vitro pour certains produits, tels que CM 7748 et 7798 qui ont une affinité nettement plus grande pour les récepteurs $\beta_1$ (coeur) que pour les récepteurs $\beta_2$ (poumon).

Tous ces produits en tant que $\beta$-bloqueurs ont une durée d'action variant de 2 h à plus de 4 h 30, lorsqu'ils sont administrés par voie intraveineuse.

Certains produits ont en plus de leur pouvoir bétalytique, une note alphalytique. In vivo (test de la noradrénaline) ce sont notamment les CM 7743, 7764, 7748, 7782, 7806, 7808, 7824, 7859, 7903 et 40038. In vitro, ces produits n'ont pas d'affinité pour le récepteur à la clonidine ($\alpha_2$). Par contre les produits CM 7744, 7806, 7808, 7824, 7874 et 7983 ont une affinité pour le récepteur au WB 4101 ($\alpha_1$).

Les CM 7748, 7782, 7798, 7859 et 7874 provoquent une bradycardie (de 10 à 20%) chez l'animal anesthésié.

Les CM 7744, 7806, 7808, 7824, 7874, 7898, 7899, 7902, 7903, 7906 et 40038 provoquent une diminution de la pression artérielle périphérique, lorsqu'ils sont administrés par voie intraveineuse chez le chien anesthésié.

L'activité antigrégante des CM 7743, 7744, 7824, 7854, 7859 et 7874 a été étudiée selon la technique de Born sur plasma riche en plaquettes d'origine humaine. L'ADP a été utilisé comme indicateur d'agrégation plaquettaire. Tous les produits testés ont inhibé la réponse plaquettaire vis-à-vis de l'ADP. La concentration requise pour inhiber à 50% le taux maximal d'agrégation varie de 45 à 100 $\mu$M.

*D'après les résultats pharmacologiques obtenus les produits selon l'invention peuvent être utilisés dans les indications thérapeutiquement suivantes:*

— Traitement des toubles pathologiques en rapport avec une hyperproduction de catécholamine: tachycardie, palpitations, extrasystoles et hypertension.

— Traitement de fond de la maladie hypertensive.

— Traitement de fond de la maladie angineuse, des sequelles d'infarctus, des troubles du rythme auriculaire et ventriculaire.

— Traitement de la migraine.

— Traitement de différents troubles neurologiques: états d'anxiété isolés ou avec localisation organique, cures de désintoxication, etc.

Ces produits peuvent être présentés sous les différentes formes destinées à l'administration orale telle que comprimés dosés de 10 à 100 mg ou à l'administration rectale telle que suppositoires dosés de 10 à 100 mg ou sous forme de préparations injectables contenant de 5 à 50 mg de principe actif.

La posologie usuelle est de 2 à 4 comprimés à 20 mg par jour mais exceptionnellement, sous surveillance médicale, elle pourra dépasser notablement ces chiffres.

On indique ci-après quelques exemples de préparations galéniques:

*Comprimés:*

| | |
|---|---|
| — CM 7748 | 20 mg |
| — cellulose microcristalline | 160 mg |
| — lactose | 172 mg |
| — stéarate de magnésium | 8 mg |
| | 360 mg |

*Suppositoires:*

| | |
|---|---|
| — CM 7898 | 40 mg |
| — Suppocire C (mélange injectable d'esters d'acides gras naturels) | qsp 3 g |
| — Labrafil 2130 C (huile de palme hydrogénée inter-estérifiée) | |

TABLEAU I

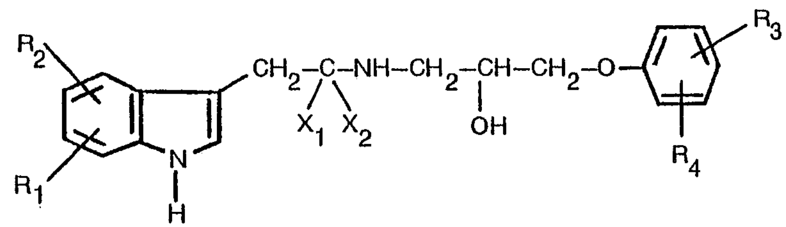

| N° de code | $X_1$ | $X_2$ | $R_1$ | $R_2$ | $R_3$ | $R_4$ | Sel ou base | Point de fusion (°C) (solvant de cristallisation) |
|---|---|---|---|---|---|---|---|---|
| 7761 | H | H | H | H | 3–$CH_3$ | H | Base | 120 (acétate d'éthyle) |
| 7783 | $CH_3$ | H | H | H | H | H | Base | 124–6 (dichlorométhane-éther isopropylique) |
| 7785 | ,, | ,, | H | H | 4–F | H | Base | 123–4 (dichlorométhane-éther isopropylique) |
| 7808 | ,, | ,, | H | H | 2–$NO_2$ | H | Base | 84–6 (dichlorométhane-éther isopropylique) |
| 7806 | ,, | ,, | H | H | 2–Cl | H | Fumarate neutre | 119–122 (éthanol-acétate d'éthyle) |
| 7744 | ,, | ,, | H | H | 2–$CH_3$ | H | chlorhydrate | 131–5 (dichlorométhane-acétate d'éthyle) |
| | | | | | | | Fumarate neutre | 185–9 (éthanol à 75%) |
| 7983 | ,, | ,, | 5'–F | H | 2–$CH_3$ | H | Fumarate acide | 140–3 (acétate d'éthyle-éther) |
| 7782 | ,, | ,, | H | H | 3–$CH_3$ | H | Fumarate neutre | 172–4 (acétate d'éthyl-éthanol) |

TABLEAU I (suite 1)

| N° de code | $X_1$ | $X_2$ | $R_1$ | $R_2$ | $R_3$ | $R_4$ | Sel ou base | Point de fusion (°C) (solvant de cristallisation) |
|---|---|---|---|---|---|---|---|---|
| 7784 | $CH_3$ | H | H | H | $4-CH_3$ | H | Fumarate acide | 180–2 (acétate d'éthyle-éthanol) |
| 7824 | $CH_3$ | H | H | H | $2-C\equiv N$ | H | Chlorhydrate | 95 (éther) |
| 7904 | $CH_3$ | H | H | H | $2-C_6H_5-C(=O)-$ | H | Fumarate neutre | 176–8 (acétate d'éthyle-éthanol) |
| 7894 | $COOCH_3$ | | H | H | $4-F$ | H | Chlorhydrate | 76–8 (éther) |
| 7897 | COOH | H | H | H | $2-CH_3$ | H | Base | 220–1 (méthanol) |
| 7803 | H | H | H | H | $4-CH_3CH_2CO-$ | H | Base | 130–1 (acétate d'éthyle-méthanol) |
| 7861 | H | H | $5'-OCH_3$ | H | $4-CH_3CH_2CO-$ | 2–Cl | Base | 113–4 (dichlorométhane-éther isopropylique) |
| 7802 | $CH_3$ | H | H | H | $4-CH_3CH_2CO-$ | H | Base | 118–120 (dichlorométhane-éther isopropylique) |
| 7743 | $CH_3$ | H | H | H | $4-CH_3CH_2CO-$ | 2–Cl | Fumarate neutre | 119–122 (acétone-méthanol) |
| 7595 | $CH_3$ | H | H | H | $4-CH_3CH_2CO-$ | 3–Cl | Base | 100–3 (dichlorométhane-éther isopropylique) |
| 7801 | $CH_3$ | H | $5'-Cl$ | H | $4-CH_3CH_2CO-$ | 2–Cl | Base | 147 (éther isopropylique-méthanol) |
| 7820 | $CH_3$ | H | $5'-OCH_3$ | $6'-OCH_3$ | $4-CH_3CH_2CO-$ | 2–Cl | Base | 127–130 (éther isopropylique) |
| 7825 | $CH_3$ | H | H | H | $4-CH_3(CH_2)_4-CO-$ | 2–Cl | Fumarate neutre | 165–6 (éthanol-éther) |

TABLEAU I (suite 2)

| N° de code | $X_1$ | $X_2$ | $R_1$ | $R_2$ | $R_3$ | $R_4$ | Sel ou base | Point de fusion (°C) (solvant de cristallisation) |
|---|---|---|---|---|---|---|---|---|
| 7844 | $CH_3$ | H | H | H | 4 — 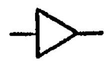 CO – | 2–Cl | Fumarate neutre | 120–4 (éther isopropylique méthanol) |
| 7845 | $C_2H_5$ | H | H | H | 4–$CH_3CH_2CO$– | 2–Cl | Fumarate neutre | 170–2 (éthanol) |
| 7809 | $CH_2OH$ | H | H | H | 4–$CH_3CH_2CO$– | 2–Cl | Base | 147–9 (éthanol) |
| 7988 | $CH_3$ | H | H | H | 4–$CH_3CH_2CH_2CO$– | 2–F | Base | 82–3 (acétate d'éthyle) |
| 7987 | $CH_3$ | H | H | H | 4–$CH_3CH_2CH_2CO$– | H | Base | 118–120 (acétate d'éthyle) |
| 7748 | H | H | H | H | 4–$CH_2CONH_2$ | H | Base | 167–8 (éthanol) |
| 7747 | H | H | H | H | 4–$CH_2COOCH_3$ | H | Base | 98–102 (acétate d'éthyle-éther isopropylique) |
| 7715 | $CH_3$ | H | H | H | 4–$CH_2CONH_2$ | H | Base | 122–4 (acétate d'éthyle-éther isopropylique) |
| 7807 | $CH_3$ | H | H | H | 4–$CH_2COOCH_3$ | H | Base | 79–83 (éther) |
| 7798 | $CH_3$ | H | H | H | 4–NH–$CO(CH_2)_2CH_3$ | 2–$COCH_3$ | Base | 122 (acétate d'éthyle-éther isopropylique) |
| 7847 | $CH_3$ | H | H | H | 4–$SO_2CH_3$ | H | Base | 119–122 (hexane-méthanol-éther isopropylique) |
| 7859 | $CH_3$ | H | 5'–Cl | H | 4–$CH_2CONH_2$ | H | Base | 155 (acétate d'éthyle) |

TABLEAU I (suite 3)

| N° de code | $X_1$ | $X_2$ | $R_1$ | $R_2$ | $R_3$ | $R_4$ | Sel ou base | Point de fusion (°C) (solvant de cristallisation) |
|---|---|---|---|---|---|---|---|---|
| 7860 | $CH_3$ | H | 5'–Cl | H | $4–CH_2COOCH_3$ | H | Base | 115 (dichlorométhane-éther isopropylique) |
| 7863 | $CH_3$ | H | H | H | $4–CH_2C{\equiv}N$ | H | Fumarate acide | 130 (acétone) |
| 7896 | H | H | H | H | $4–(CH_2)_2NHCOOC_2H_5$ | H | Base | 101–2 (dichlorométhane-éther isopropylique) |
| 7895 | H | H | H | H | $4–O–(CH_2)_2NHCOOC_2H_5$ | H | Base | 111–2 (dichlorométhane-éther isopropylique) |
| 40185 | $C_2H_5$ | H | H | H | $2–CH_3$ | H | Fumarate neutre | 168–171 (éthanol) |
| 40269 | $CH_3$ | H | H | H | $4–(CH_2)_2NHCOOC_2H_5$ | H | Base | Huile |
| 40405 | $CH_3$ | H | H | H | $4–O–(CH_2)_2NHCOOC_2H_5$ | H | Base | Huile |
| 40038 | $CH_3$ | H | 5–F | H | H | H | Base | 73–4 (hexane-dichlorométhane) |

TABLEAU II

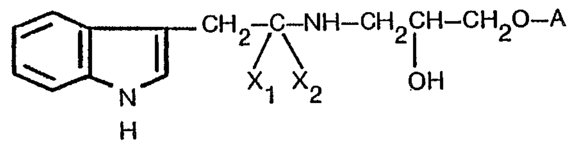

| N° de code | X1 | X2 | A | Sel ou base | Point de fusion (°C) (solvant de cristallisation) |
|---|---|---|---|---|---|
| 7701 | $CH_3$ | H | 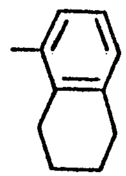 | Chlorhydrate | 168–169 (méthanol-éther isopropylique) |
| 7862 | $CH_3$ | H | 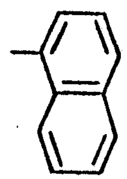 | Fumarate neutre | 128–130 (acétone-méthanol-éther) |
| 7852 | $CH_3$ | $CH_3$ | 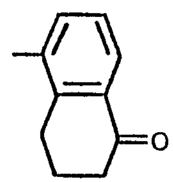 | Base | 76–78 (dichlorométhane-éther isopropylique) |

TABLEAU II (suite)

| N° de code | $X_1$ | $X_2$ | A | Sel ou base | Point de fusion (°C) (solvant de cristallisation) |
|---|---|---|---|---|---|
| 7853 | $CH_3$ | $CH_3$ | 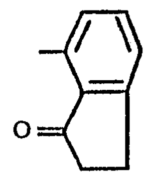 | Fumarate acide | 168–171 (méthanol-acétate d'éthyle) |
| 7992 | $CH_3$ | $CH_3$ | 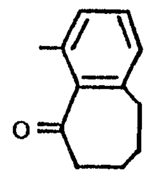 | Fumarate acide | 165–166 (acétone-méthanol) |
| 7874 | $CH_3$ | H | 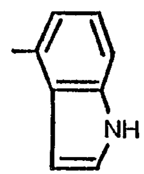 | Fumarate acide | 170 (méthanol) |
| | | | | Fumarate neutre | 187 (éthanol) |
| 40441 | $CH_3$ | $CH_3$ | 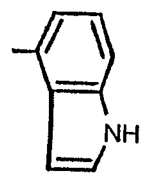 | Fumarate neutre | 177 (éthanol) |

TABLEAU III

| N° CM | $DI_{50}$ ISOPRENALINE mg/kg | | | $DI_{50}$ NORADRENALINE mg/kg | $CE_3$ 50 du DEPLACEMENT du $^3H$ DIHYDROALPRENOLOL en nM | | $CE_{50}$ du DEPLACEMENT de la $^3H$ CLONIDINE en nM | $CE_{50}$ du DEPLACEMENT du $^3H$ WB 4101 en nM |
|---|---|---|---|---|---|---|---|---|
| | $\beta_1-$ | $\beta_2-$ | $\beta_1-\beta_2-$ | $\alpha-$ | Coeur ($\beta_1$) | Poumon ($\beta_2$) | | |
| 7743 | 1 à 3 | $\geqslant 3$ | $\beta_1 \geqslant \beta_2$ | 3 | 5.300 | 6.300 | 26.500 | 2.200 |
| 7744 | 0,1 | $\geqslant 0,3$ | $\beta_1 > \beta_2$ | 0,1 à 1 | 31 | 52 | 20.500 | 500 |
| 7748 | 1 à 3 | >3 | $\beta_1 > \beta_2$ | 1 | 15.000 | 57.000 | 29.500 | 2.700 |
| 7782 | 0,1 à 0,3 | $\geqslant 3$ | $\beta_1 > \beta_2$ | 0,3 | 53 | 61,5 | 30.000 | 1.460 |
| 7798 | 1 | $\geqslant 3$ | $\beta_1 > \beta_2$ | >3 | 550 | 3.700 | | |
| 7806 | 0,3 | $\geqslant 0,1$ | $\beta_1 \leqslant \beta_2$ | 0,3 | 27,5 | 12 | 12.000 | 310 |
| 7808 | 0,3 | 1 | $\beta_1 > \beta_2$ | 0,3 | 28,5 | 24 | 25.000 | 320 |
| 7824 | 0,3 | $\geqslant 0,3$ | $\beta_1 \geqslant \beta_2$ | 1 | 13,5 | 10 | 9.500 | 110 |
| 7859 | 0,3 à 1 | $\geqslant 3$ | $\beta_1 > \beta_2$ | 0,3 à 3 | 625 | 1000 | 19.000 | 2.300 |
| 7874 | 0,01 | 0,01 à 0,3 | $\beta_1 \geqslant \beta_2$ | >0,03 | 4 | 8,2 | 20.000 | 215 |
| 7898 (isomère SS) | 0,1 à 0,3 | 0,3 à 1 | $\beta_1 \geqslant \beta_2$ | >3 | 51 | 64 | | |

TABLEAU III (suite)

| N° CM | $DI_{50}$ ISOPRENALINE mg/kg | | | $DI_{50}$ NORADRENALINE mg/kg | $CE_3$ 50 du DEPLACEMENT du $^3H$ DIHYDROALPRENOLOL en nM | | $CE_{50}$ du DEPLACEMENT de la $^3H$ CLONIDINE en nM | $CE_{50}$ du DEPLACEMENT du $^3H$ WB 4101 en nM |
|---|---|---|---|---|---|---|---|---|
| | $\beta_1$ – | $\beta_2$ – | $\beta_1$–$\beta_2$– | $\alpha$ – | Coeur ($\beta_1$) | Poumon ($\beta_2$) | | |
| 7899 (isomère SR) | 0,3 | 0,3 à 1 | $\beta_1 \geq \beta_2$ | >3 | 135 | 82 | | |
| 7902 (isomère RR) | ≥3 | >3 | $\beta_1 < \beta_2$ | >3 | 7400 | 3800 | | |
| 7903 (isomère RS) | 3 | 1 | $\beta_1 < \beta_2$ | 3 | 2050 | 2000 | 29.500 | 1.900 |
| 7904 | ≤0,1 | 0,1 | $\beta_1 \geq \beta_2$ | >0,3 | 7,9 | 14 | | |
| 7906 (racémique) | 0,3 à 1 | 1 | $\beta_1 \geq \beta_2$ | >3 | 215 | 155 | 41.000 | 1.100 |
| 7983 | 0,3 | >0,3 | $\beta_1 > \beta_2$ | >0,3 | 19 | 47 | >100.000 | 600 |
| 40038 | 0,1 | >0,3 | $\beta_1 > \beta_2$ | 0,1 | 34 | 29 | | |
| Propanolol | 0,3 à 1 | 0,3 | $\beta_1 = \beta_2$ | | 17,5 | 28,6 | >100,000 | 10.500 |
| 7854 | 0,01 – 0,1 | 0,02 – 0,1 | $\beta_1 \geq \beta_2$ | 0,1 ou RAS | 16 | 3,7 | 6.000 | 320 |

**Revendications**

1. Produits chimiques de formule générale

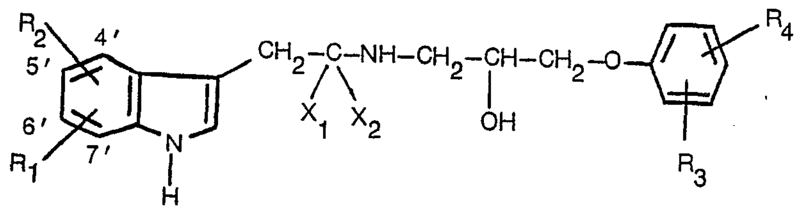

(I)

dans laquelle $R_1$ et $R_2$ désignent chacun indépendamment un atome d'hydrogène ou un atome d'halogène, un groupe alkyle en $C_1$—$C_5$, un groupe alcoxy en $C_1$—$C_5$, occupant l'une des positions 4′ à 7′ du noyau indole; $R_3$ et $R_4$ considérés indépendamment désignent un atome d'hydrogène, un alkyle en $C_1$—$C_5$, un alcoxy en $C_1$—$C_5$, un atome d'halogène, un groupe nitro, un groupe acycle $R_5CO$, un groupe alkylthio $R_5S$ ou alkylsulfinyl $R_5SO$ ou alkylsulfonyl $R_5SO_2$ dans lesquels $R_5$ désigne un groupe alkyle en $C_1$—$C_5$ ou cyclopropyle ou phényle, un groupe $-(CH_2)_nCOOR_6$, $(CH_2)_nCONH$-$R_6$ ou $CH_2CONH_2$ ou $(CH_2)_nNHCOOR_6$ ou $(CH_2)_nNHCOR_6$ dans lesquels n représente un entier de 0 à 2 et $R_6$ désigne un groupe alkyle en $C_1$—$C_5$; ou $R_3$ et $R_4$ pris ensemble peuvent former un cycle comportant éventuellement un hétéroatome de façon à constituer, avec le cycle benzénique auquel ils sont liés, un noyau bicyclique choisi parmi les noyaux suivants:

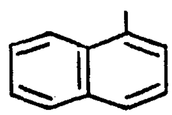
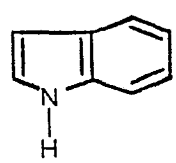
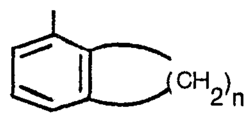

n = 3 à 5

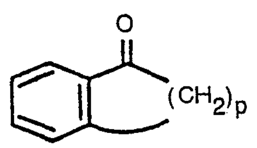
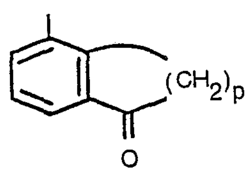

p = 2 à 4

$X_1$ représente un atome d'hydrogène, un groupe alkyle en $C_1$—$C_5$, un groupe hydroxyméthyle ou encore un groupe $COOR_7$ dans lequel $R_7$ représente l'hydrogène ou un groupe alkyle en $C_1$—$C_5$; $X_2$ représente l'hydrogène ou dans le cas où

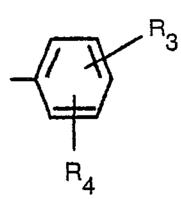

représente un noyau bicyclique autre que naphtyle $X_2$ peut être un groupe méthyle, ainsi que les sels desdits produits avec des acides minéraux ou organiques pharmaceutiquement acceptables.

2. Produits chimiques selon la revendication 1, caractérisés en ce qu'ils sont sous la forme d'un isomère.

3. Procédé de préparation d'un produit selon la revendication 1, caractérisé en ce que l'on effectue les opérations suivantes:

— on fait réagir l'épichlorhydrine sur un phénol de formule

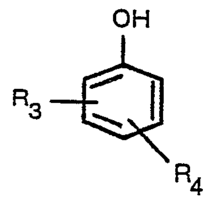

ladite réaction étant effectuée dans un solvant anhydre et en présence d'un agent alcalin;

— puis on fait réagir sur le produit obtenu une amine de formule

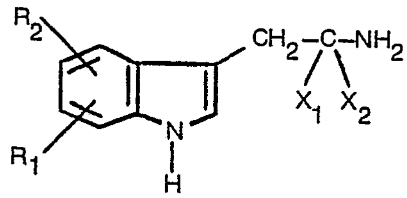

la réaction étant effectuée au sein d'un solvant anhydre.

4. Procédé de préparation d'un produit selon la revendication 1 dans lequel $X_1$ est $CH_3$ et $X_2$ est H, caractérisé en ce que l'on fait réagir un acétonyl indole de formule

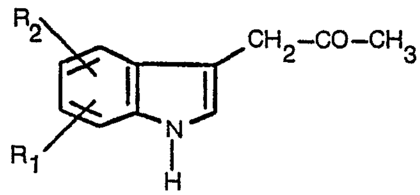

sur un phénoxy-1 amino-3 propanol-2 de formule

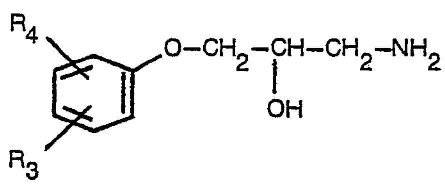

la réaction ayant lieu au sein d'un solvant à température voisine de l'ambiante, de façon à former l'imine que l'on traite ensuite à l'aide d'un agent réducteur tel que le borohydrure de sodium.

5. Médicaments actifs notamment sur le système cardiovasculaire, caractérisés en ce qu'ils contiennent comme principe actif une quantité efficace d'au moins un produit nouveau selon l'une des revendications 1 et 2.

6. Médicaments selon la revendication 5, caractérisés en ce qu'ils sont conditionnés pour une administration orale ou rectale à raison de 10 à 100 mg de substance active.

7. Médicaments selon de revendication 5, caractérisés en ce qu'ils sont conditionnés sous forme injectable à raison de 5 à 50 mg de substance active.

**Claims**

1. Chemical products of general formula:

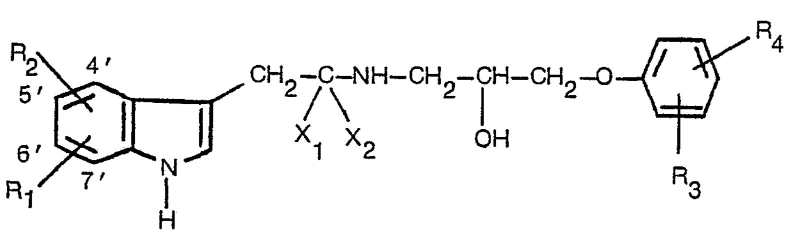

(I)

in which

— $R_1$ and $R_2$ each designate, independently, an atom of hydrogen or an atom of halogen, a lower $C_1$—$C_5$ alkyl group, a $C_1$—$C_5$ alkoxy group, occupying one of the positions 4′ to 7′ of the indole ring;

— $R_3$ and $R_4$ considered independently designate an atom of hydrogen, a $C_1$—$C_5$ alkyl, a $C_1$—$C_5$ alkoxy, an atom of halogen, a nitro group, an acyl $R_5CO$ group, an alkylthio $R_5S$ group or alkylsulfinyl $R_5SO$ or alkylsulfonyl $R_5SO_2$ group in which $R_5$ designates a $C_1$—$C_5$ alkyl group or cyclopropyl or phenyl group, a $(CH_2)_n$ $COOR_6$ group, $(CH_2)_n$ CONH—$R_6$ or $CH_2CONH_2$ or $(CH_2)_n$ $NHCOOR_6$ or $(CH_2)_n$ $NHCOR_6$ group in which $n$ represents an integer from 0 to 2 and $R_6$ designates a $C_1$—$C_5$ alkyl group;

— or $R_3$ and $R_4$ taken together may form a cycle possibly comprising the heteroatom so as to constitute, with the benzene cycle to which they are bonded, a bicyclic ring selected from the following rings:

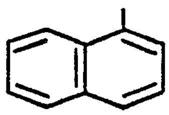

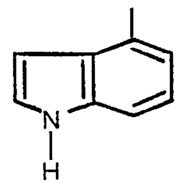

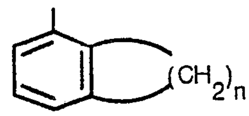

$n = 3$ to 5

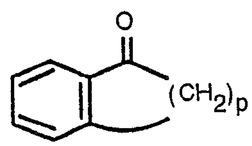

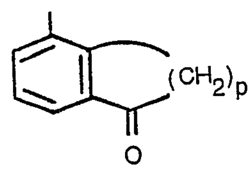

$p = 2$ to 4

— $X_1$ represents an atom of hydrogen, a $C_1$—$C_5$ alkyl group, a hydroxymethyl group or a $COOR_7$ group in which $R_7$ represents hydrogen or a $C_1$—$C_5$ alkyl group;

— $X_2$ represents hydrogen or in the case of

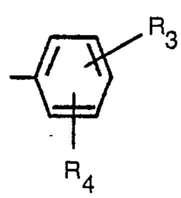

representing a bicyclic ring other than naphthyl, $X_2$ may be a methyl group, as well as the salts of said products with pharmaceutically acceptable inorganic or organic acids.

2. Chemical products according to claim 1, characterized in that they are in the form of an isomer.

3. Process for preparing a product according to claim 1, characterized in that it comprises the following steps of:

— reacting epichlorohydrine on a phenol of formula

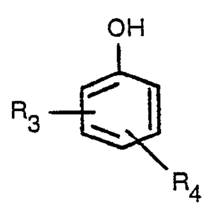

said reaction being effected in an anhydrous solvent and in the presence of an alkaline agent,

— then reacting on the product obtained an amine of formula:

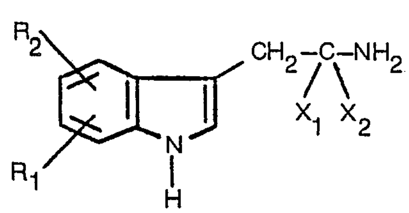

the reaction being effected within an anhydrous solvent.

4. Process for preparing a product according to claim 1, in which $X_1$ is $CH_3$ and $X_2$ is H, characterized in that an acetonyl indole of formula

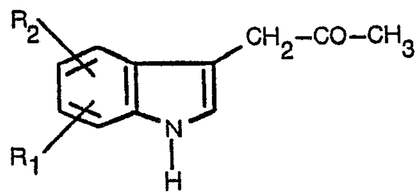

is reacted on a phenoxy-1 amino-3 propanol-2 of formula

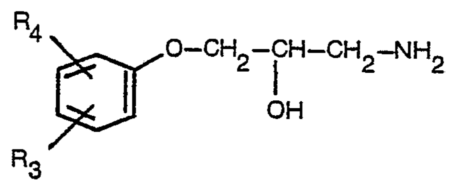

the reaction taking place within a solvent at temperature close to ambient temperature, so as to form the imine which is then treated with the aid of a reducing agent such as sodium borohydride.

5. Drugs particularly active on the cardiovascular system, characterized in that they contain as active ingredient an effective quantity of at least one novel product according to any one of claims 1 and 2.

6. Drugs according to claim 5, characterized in that they are prepared for oral or rectal administration at a rate of 10 to 100 mg of active substance.

7. Drugs according to claim 5, characterized in that they are prepared in injectable form at a rate of 5 to 50 mg of active substance.

**Patentansprüche**

1. Chemische Produkte der allgemeinen Formel

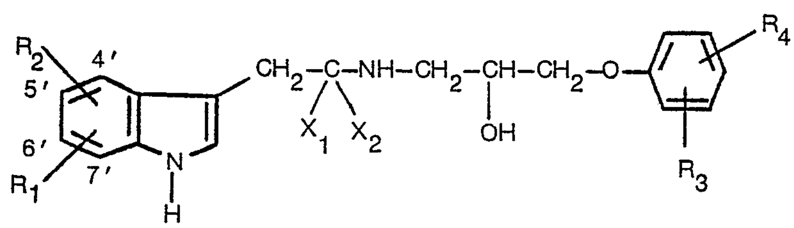

(I)

worin $R_1$ une $R_2$ jeweils unabhängig voneinander ein Wasserstoffatom oder ein Halogenatom, eine $C_1$—$C_5$-Alkylgruppe, eine $C_1$—$C_5$-Alkoxygruppe, die eine der Positionen 4′ bis 7′ des Indolkerns einnehmen, bedeuten; $R_3$ und $R_4$, unabhängig voneinander betrachtet, ein Wasserstoffatom, $C_1$—$C_5$-Alkyl, $C_1$—$C_5$-Alkoxy, ein Halogenatom, eine Nitrogruppe, eine Acylgruppe $R_5CO$, eine O Alkylthiogruppe $R_5S$ oder Alkylsulfinylgruppe $R_5SO$ oder Alkylsulfonylgruppe $R_5SO_2$ bedeuten, worin $R_5$ eine $C_1$—$C_5$-Alkylgruppe oder Cyclopropyl oder Phenyl, eine $—(CH_2)_n—$ $COOR_6—$, $—(CH_2)_nCONH—R_6—$ oder $—CH_2CONH_2—$ oder $—(CH_2)_n—$ $NHCOOR_6—$ oder $—(CH_2)_nNHCOR_6—$ gruppe darstellt, in denen n eine ganze Zahl von 0 bis 2 ist und $R_6$ für eine $C_1$—$C_5$-Alkylgruppe steht; oder $R_3$ und $R_4$ zusammen einen gegebenenfalls ein Heteroatom umfassenden Ring bilden können, sodaß sie mit dem Benzolring, an den sie gebunden sind, einen bicyclischen Kern bilden, der unter den folgenden Kernen ausgewählt ist:

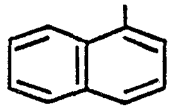
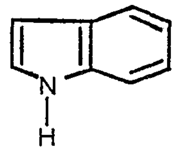
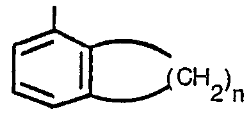

n = 3 bis 5

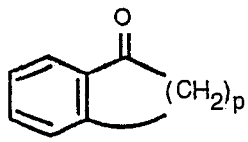
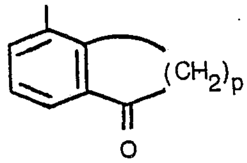

p = 2 bis 4

$X_1$ ein Wasserstoffatom, eine $C_1$—$C_5$-Alkylgruppe, eine Hydroxymethylgruppe oder auch eine $COOR_7$-Gruppe, worin $R_7$ für Wasserstoff oder eine $C_1$—$C_5$-Alkylgruppe steht, darstellt; $X_2$ Wasserstoff darstellt oder für den Fall, daß

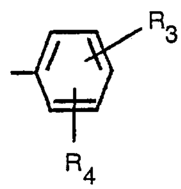

einen bicyclischen Kern, außer Naphthyl, bedeutet, $X_2$ eine Methylgruppe sein kann, sowie die Salze der genannten Produkte mit den pharmazeutisch akzeptablen Mineral- oder organischen Säuren.

2. Chemische Produkte nach Anspruch 1, dadurch gekennzeichnet, daß sie in Form eines Isomeren vorliegen.

3. Verfahren zur Herstellung eines Produktes nach Anspruch 1, dadurch gekennzeichnet, daß die folgenden Schritte durchgeführt werden:

— Epichlorhydrin auf ein Phenol der Formel

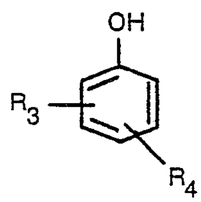

einwirken gelassen wird, wobei die Reaktion in einem wasserfreien Lösungsmittel und in Gegenwart eines alkalischen Mittels durchgeführt wird;

— danach auf das erhaltene Produkt ein Amin der Formel

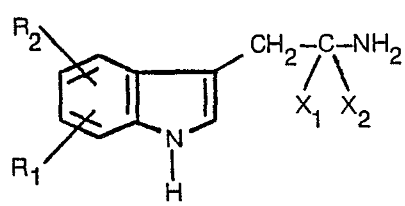

einwirken gelassen wird, wobei die Reaktion in einem wasserfreien Lösungsmittel durchgeführt wird.

4. Verfahren zur Herstellung eines Produktes nach Anspruch 1, worin $X_1$ $CH_3$ und $X_2$ H ist, dadurch gekennzeichnet, daß ein Acetonylindol der Formel

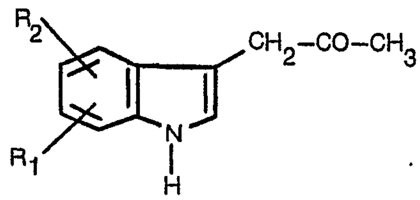

auf ein 1-Phenoxy-3-amino-2-propanol der Formel

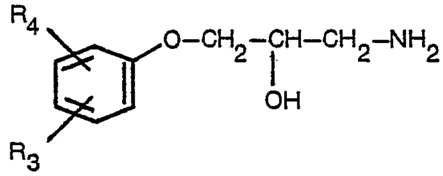

einwirken gelassen wird, wobei die Reaktion inmitten eines Lösungsmittels mit einer Temperatur nahe der Umgebungstemperatur stattfindet, sodaß das Imin gebildet wird, welches danach mit einem Reduktionsmittel, wie Natriumborhydrid, behandelt wird.

5. Medikamente, die insbesondere auf das Kreislaufsystem wirken, dadurch gekennzeichnet, daß sie als Hauptwirkstoff eine wirkungsvolle Menge von zumindest einem neuen Produkt nach einem der Ansprüche 1 und 2 enthalten.

6. Medikamente nach Anspruch 5, dadurch gekennzeichnet, daß sie für oral oder rektale Verabreichung zu 10 bis 100 mg Aktivstoff konditioniert sind.

7. Medikamente nach Anspruch 5, dadurch gekennzeichnet, daß sie in injizierbarer Form zu 5 bis 50 mg Aktivstoff konditioniert sind.